# EUROPEAN PATENT SPECIFICATION

(11) **EP 2 321 306 B1**
(45) Date of publication and mention of the grant of the patent: **18.01.2012**
(21) Application number: 09772403.3
(22) Date of filing: 29.06.2009
(51) Int. Cl.: C07D 413/08, A61K 31/422, A61P 31/04

(54) **3-CYANOPYRROLIDINYL-PHENYL-OXAZOLIDINONES AS ANTIBACTERIAL AGENTS**
3-CYANOPYRROLIDINYLPHENYLOXAZOLIDINONE ALS ANTIBAKTERIELLE MITTEL
3-CYANOPYRROLIDINYL-PHÉNYL-OXAZOLIDINONES COMME AGENTS ANTIBACTÉRIENS

(30) Priority: 01.07.2008 EP 08159446
(43) Date of publication of application: 18.05.2011
(73) Proprietor: Ferrer Internacional, S.A., 08028 Barcelona (ES)
(72) Inventor: CANO, Montserrat, E-08691 Monistrol (Barcelona) (ES); PALOMER, Albert, E-08014 Barcelona (ES); GUGLIETTA, Antonio, E-08750 Molins de Rei (Barcelona) (ES)
(74) Representative: ZBM Patents
(86) International application number: PCT/EP2009/058125
(87) International publication number: WO 2010/000703

(56) References cited:
- WO-A-96/13502
- WO-A-2005/054234
- DU YU ET AL: "Synthesis and antibacterial activity of linezolid analogUES" BIOORGANIC & MEDICINAL CHEMISTRY LETTERS, PERGAMON, ELSEVIER SCIENCE, GB, vol. 12, 1 January 2002 (2002-01-01), pages 857-859, XP002245432 ISSN: 0960-894X

## Description

### TECHNICAL FIELD

This invention is directed to antimicrobial oxazolidinone compounds which are active against Gram-positive and some Gram-negative bacteria, showing specifically a potent activity against linezolid-resistant (LNZ-R) strains of Gram-positive bacteria and more specifically against Gram-positive pathogenic respiratory bacteria.

### BACKGROUND ART

Oxazolidinones are Gram-positive antimicrobial agents. Oxazolidinones bind to the 50S subunit of the prokaryotic ribosome, preventing formation of the initiation complex for protein synthesis. This is a novel mode of action. Other protein synthesis inhibitors either block polypeptide extension or cause misreading of mRNA. Linezolid (N-[[(5S)-3-[3-fluoro-4-(4-morpholinyl)phenyl]-2-oxo-5-oxazolidinyl]methyl]acetamide), US5688792, is the first approved antimicrobial oxazolidinone for clinical use in the United States and elsewhere. The structural formula of linezolid is:

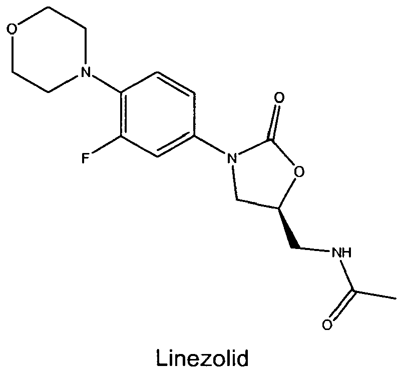

Linezolid minimal inhibitory concentrations (MICs) vary slightly with the test mode, laboratory, and significance attributed to thin hazes of bacterial survival, but all workers find that the susceptibility distributions are narrow and unimodal with MIC values between 0.5 and 4 µg/mL for *streptococci*, *enterococci* and *staphylococci.* Full activity is retained against Gram-positive *cocci* resistant to other antibiotics, including methicillin-resistant *staphylococci* and vancomycin-resistant *enterococci.* MICs are 2-8 µg/mL for *Moraxella*, *Pasteurella* and *Bacteroides* spp. but other Gram-negative bacteria are resistant as a result of endogenous efflux activity as well as the intake presented by Gram-negative bacteria outer membrane cell. Linezolid is indicated for the treatment of adult patients with the following infections: vancomycin-resistant *Enterococcus faecium* infections, including concurrent bacteremia; nosocomial pneumonia; complicated skin and skin structure infections; community-acquired pneumonia, including concurrent bacteremia; diabetic foot infections; and uncomplicated skin and skin structure infections.

Unfortunately, some Gram-positive bacteria such as Staphylococcus *aureus* (LNZ-R 432), *Haemophylus influenzae* (ATCC 49247), *Bacteroides fragilis* (ATCC 25285), *Moraxella catarrhalis* (HCl-78), and *Enterococcus faecium* (LNZ-R) show an important resistance to linezolid.

Other oxazolidinones are also known for the treatment of microbial infections. For instance, WO 2005/054234 describes piperidino substituted phenyloxazolidinones for the treatment or prevention of Gram-positive or Gram negative microbial infections, including those which result from multi-resistant strains for instance, linezolid-resistant strains.

International patent application WO 96/13502 discloses phenyl oxazolidinones having a multisubstituted azetidinyl or pyrrolidinyl moiety. These compounds are useful antimicrobial agents, which are effective against a number of human and veterinary pathogens, particularly aerobic gram-positive bacteria, including some activity against multiply-resistant staphylococci, enterococci and streptococci.

Although there are known some oxazolidinones which have some activity against linezolid-resistant Gram-positive bacteria, there continue being the need of new oxazolidinone compounds active against these strains, since some of them are the origin of severe and sometimes fatal infections such as sepsis and septic shock. There is also a need for improved agents against Gram-positive pathogenic respiratory bacteria, like *Streptococcus pneumoniae, Haemophylus influenzae,* and *Moraxella catarrhalis.*

### SUMMARY OF THE INVENTION

Surprisingly the compounds of the present application are potent active antimicrobial agents showing a relevant activity against LNZ-R Gram-positive bacteria and more specifically against Gram-positive pathogenic respiratory bacteria. Differential characteristic properties of the compounds of the present invention versus linezolid indicate the potential use thereof in severe infections that cannot be properly treated with linezolid.

In a first aspect the present invention refers to a compound of formula (I), 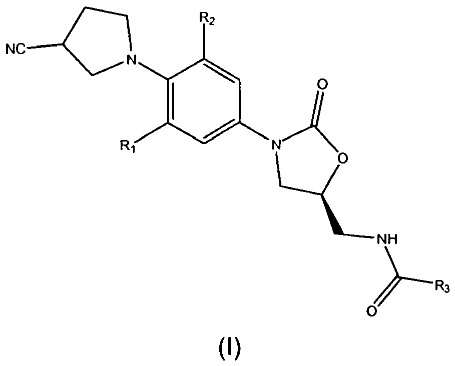 in free or pharmaceutically acceptable salt, solvate, hydrate, or enantiomeric form, wherein:
R₁ and R₂ are radicals identical or different and are independently selected from hydrogen and fluorine;
R₃ is a linear or branched (1-6C)alkyl group optionally substituted by a group selected from fluorine, hydroxy and OR₄; and
R₄ is a linear or branched (1-6C)alkyl group.

In a second aspect the present invention refers to a process for preparing a compound of formula (I) as defined in the first aspect of the invention in free or pharmaceutically acceptable salt, solvate, hydrate, or enantiomeric form that comprises:
(i) reacting an intermediate of formula (II), 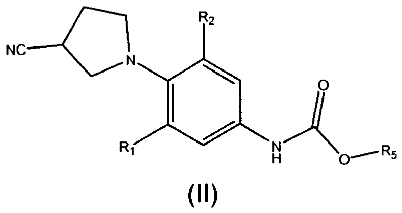 wherein R₁ and R₂ are as defined above and R₅ is selected from linear or branched (1-6C)alkyl and benzyl optionally phenyl-substituted by up to three linear or branched (1-6C)alkyl groups, with an intermediate of formula (III), 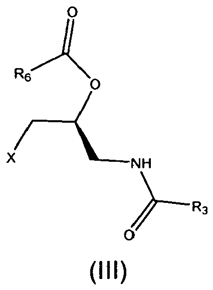 wherein R₃ is as defined above, R₆ is a linear or branched (1-6C)alkyl group, and X is a halogen atom; and
(ii) recovering the resultant compound of formula (I) in free or pharmaceutically acceptable salt, solvate, hydrate, or enantiomeric form.

In a third aspect the present invention refers to a pharmaceutical composition comprising a therapeutically effective amount of the compound of general formula (I) according to the first aspect of the invention, together with the appropriate amounts of pharmaceutical excipients or carriers.

In a fourth aspect the present invention refers to a compound of formula (I) according to the first aspect of the invention, for use as a medicament.

In an fifth aspect the present invention refers to the use of a compound of formula (I) according to the first aspect of the invention for the manufacture of a medicament for the treatment of bacterial infections in an animal or human. This aspect may also be formulated as a compound of formula (I) according to the first aspect of the invention for use in the treatment of bacterial infections.

Another object of this invention is to provide novel methods to treat a mammal, including a human, suffering from a bacterial infection by administering a therapeutically effective amount of a compound of formula (I) in free or pharmaceutically acceptable salt, solvate, hydrate, or enantiomeric form.

### DETAILED DESCRIPTION OF THE INVENTION

The term "pharmaceutically acceptable salts" used herein encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids, and the like, and any salt formed from organic and inorganic bases, such as the alkali metal and alkaline earth metal salts, especially the sodium and potassium salts, ammonium salts and salts of amines, including lower alkylated amines, such as methylamine, ethylamine, trimethylamine and the like, hydroxyloweralkylamines, such as ethanolamine and diethanolamine, and heterocyclic amines, such as morpholine and piperazine.

In a preferred embodiment, the present invention refers to a compound according to the first aspect of the invention wherein R₁ is fluorine, R₂ is selected from fluorine and hydrogen, and R₃ is methyl.

Preferably, the compound according to the first aspect of the invention is selected from the group consisting of:
*N*-{(5S)-3-[3-fluoro-4-(3-cyanopyrrolidin-1-yl)phenyl]-2-oxo-5-oxazolidinylmethyl}acetamide;
*N*-{(5*S*)-3-[3,5-difluoro-4-(3-cyanopyrrolidin-1-yl)phenyl]-2-oxo-5-oxazolidinylmethyl}acetamide;
*N*-{(5S)-3-[3-fluoro-4-(3(*R*)-cyanopyrrolidin-1-yl)phenyl]-2-oxo-5-oxazolidinylmethyl}acetamide;
*N*-{(5*S*)-3-[3,5-difluoro-4-(3(*R*)-cyanopyrrolidin-1-yl)phenyl]-2-oxo-5-oxazolidinylmethyl}acetamide;
N-{(5S)-3-[3-fluoro-4-(3(*S*)-cyanopyrrolidin-1-yl)phenyl]-2-oxo-5-oxazolidinylmethyl}acetamide; and
*N*-{(5*S*)-3-[3,5-difluoro-4-(3(*S*)-cyanopyrrolidin-1-yl)phenyl]-2-oxo-5-oxazolidinylmethyl}acetamide.

The compounds of general formula (I) may be prepared by
(i) reacting an intermediate of formula (II), 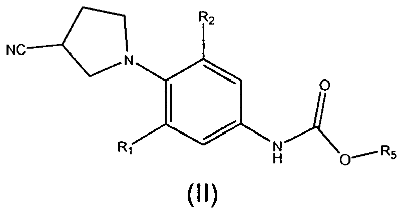 wherein R₁ and R₂ are as defined above and R₅ is selected from linear or branched (1-6C)alkyl and benzyl optionally phenyl-substituted by up to three linear or branched (1-6C)alkyl groups, with an intermediate of formula (III), 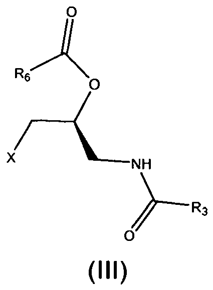 wherein R₃ is as defined above, R₆ is a linear or branched (1-6C)alkyl group, and X is a halogen atom, in an inert solvent and in the presence of a strong basic catalyst; and
(ii) recovering the resultant compound of formula (I) in free or pharmaceutically acceptable salt, solvate, hydrate, or enantiomeric form.

Preferably R₅ is benzyl, R₆ is methyl and X is bromine.

Inert solvents in step (i) are preferably aprotic solvents. Suitable aprotic solvents are polar ethers such as, for example, tetrahydrofuran, methyltetrahydrofuran, dioxane, tert-butylmethylether, or dimethoxyethylether, or amides such as, for example, dimethylformamide, or lactams such as, for example, N-methylpyrrolidone, and mixtures thereof. Suitable solvents are also mixtures of such aprotic solvents and alcohols such as, for example, methanol or ethanol.

Examples of strong basic catalysts include hydroxides such as lithium hydroxide, sodium hydroxide, and potassium hydroxide, alkoxides, such as lithium *tert*-butoxide, sodium *tert*-butoxide, and potassium *tert*-butoxide, alkyllithiums such as *tert*-butyllithium, n-butyllithium, and methyllithium, dialkylamides such as lithium diisopropylamide, disilylamides such as lithium hexamethyldisilazide, potassium hexamethyldisilazide, and sodium hexamethyldisilazide, and hydrides such as lithium hydride, sodium hydride, and potassium hydride.

Useful processes for recovering the resultant compounds in step (ii) include conventional methods known to the person skilled in the art such as crystallization and chromatographic processes, resolution of racemic forms by chromatographic separation using a chiral stationary phase, and also processes involving fractional crystallization. This can, in particular, involve the separation of individual enantiomers, for example, diastereoisomeric salts formed with chiral acids, for example (+)-tartaric acid, (-)-tartaric acid, or (+)-1 0-camphorsulfonic acid.

The compounds of the present invention are useful antimicrobial agents, effective against a number of human and veterinary microorganisms. In a preferred embodiment, the compounds of the present invention are effective against an infection produced by linezolid-resistant strain. In another preferred embodiment, the compounds of the present invention are effective against an infection produced by Gram-positive pathogenic respiratory bacteria Some non limitative examples of these microorganisms are *Staphylococcus aureus*, *Streptococcus pneumoniae*, *Haemophylus influenzae*, *Bacteroides fragilis*, *Moraxella catarrhalis*, and *Enterococcus faecium*. As it is illustrated in the Examples 4 and 6, the compounds of the present invention are more active against linezolid-resistant strains than both linezolid and the closest structurally substituted oxazolidinone of the state of the art. They are also more active against Gram-positive pathogenic respiratory bacteria than both linezolid and the closest structurally substituted oxazolidinone of the state of the art.

The compounds of the present invention can be normally formulated in accordance with standard pharmaceutical practice as a pharmaceutical composition.

The pharmaceutical compositions of this invention may be administered in standard manner for the disease condition that it is desired to treat, for example by oral, parenteral, inhalatory, rectal, transdermal or topical administration. For these purposes the compounds of this invention may be formulated by means known in the art in the form of, for example, tablets, capsules, syrups, aqueous or oily solutions or suspensions, emulsions, dispersible powders, inhalatory solutions, suppositories, ointments, creams, drops and sterile aqueous or oily solutions or suspensions for injection and the like. The pharmaceutical compositions may contain flavoring agents, sweeteners, etc. in suitable solid or liquid carriers or diluents, or in a suitable sterile media to form suspensions or solutions suitable for intravenous, subcutaneous or intramuscular injection. Such compositions typically contain from 1 to 40%, preferably 1 to 10% by weight of active compound, the remainder of the composition being pharmaceutically acceptable carriers, diluents, solvents and the like.

The compounds of formula (I) are administered in an amount of 0.1 to 100 mg/kg of body weight/day, preferably 1 to 50 mg/kg of body weight/day. The compounds and compositions of the present invention are useful in the treatment of conditions such as nosocomial pneumoniae, community acquired pneumoniae, caused by methicillin-resistant *Staphylococcus aureus* (MRSA), including concurrent bacteremia, penicillin resistance and sensitive *Streptococcus pneumoniae*, diabetic foot infections and skin and skin structure infections, and all other infections caused by bacteria sensitive to the compounds described in the invention. The compounds of the present invention are effective against a number of human or animal pathogens, clinical isolates, including vancomycin-resistant organisms, methicillin-resistant organisms, and LNZ-R organisms.

Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples are provided by way of illustration, and are not intended to be limiting of the present invention.

### EXAMPLES

### Example 1: N-{(5S)-3-[3-fluoro-4-(3-cyanopyrrolidin-1-yl)phenyl]-2-oxo-5-oxazolidinylmethy}acetamide

### a) 3-Fluoro-4-(3-cyanopyrrolidin-1-yl)nitrobenzene

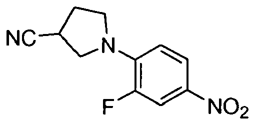

3-Carbonitrile pyrrolidine (133 mg) and potassium carbonate (268 mg) were dissolved in dimethylformamide (1 mL), and 3,4-difluoronitrobenzene (198 mg) added. The mixture was stirred at room temperature under nitrogen for 20 hours. Dichloromethane (DCM) was added to the reaction mixture and washed with water, brine and dried (magnesium sulfate). The residue was purified by column chromatography (11 g silica gel, DCM) to get 249 mg of title product (Yield = 80%).
HPLC (t, %): 8.04 min, 100%.
MS(ESI) *m*/*z* = 236 (M+1)
¹H NMR (400 MHz, ppm, CDCl₃): 2.38 (2H, m), 3.27 (1H, m), 3.67 (1H, m), 3.77 (1H, m), 3.89 (2H, m), 6.57 (1H, t, J = 9.2 Hz), 7.92 (2H, m)

### b) 3-Fluoro-4-(3-cyanopyrrolidin-1-yl)phenylamine

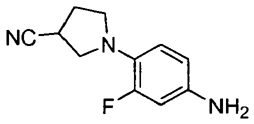

3-Fluoro-4-[3-cyanopyrrolidinyl]nitrobenzene (220 mg) was dissolved in ethanol
(20 mL) and treated with tin chloride (SnCl₂.2H₂0, 1.5 g). The mixture was stirred and heated to reflux under nitrogen for 6 hours. Aqueous sodium bicarbonate and DCM were added, the organic layer separated and the aqueous layer extracted with DCM. The combined organic layers were dried and concentrated to give title product (141 mg. Yield = 73%).
HPLC (t, %): 6.41 min, 100%.
MS(ESI) *m*/*z* = 206 (M+1)
¹H NMR (400 MHz, ppm, CDCl₃): 2.68 (2H, m), 3.21 (1H, m), 3.30 (2H, m), 3.35 (1H, m), 3.49 (1H, m), 6.45 (2H, m), 6.59 (1H, t, J = 8 Hz)

### c) 3-Fluoro-4-(3-cyanopyrrolidin-1-yl)phenylcarbamic acid benzyl ester

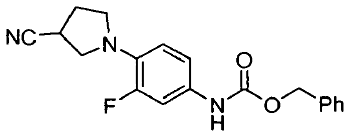

3-Fluoro-4-[3-cyanopyrrolidinyl]phenylamine (120 mg) was dissolved in acetone (4 mL) and cooled to 0°C. Sodium hydrogen carbonate (196 mg, 4 eq) in water (2 mL) was added, followed by benzyl chloroformate (199 mg, 2 eq) over 30 minutes. The mixture was stirred and the temperature allowed to rise to ambient over 3 hours. DCM was added and the organic layer separated, and washed with water and brine. The combined organic layers were dried over magnesium sulfate and concentrated. The residue was purified by column chromatography (10 gr silica gel) eluting with DCM and DCM/MeOH 98/2 to give 185 mg of title product (Yield = 93%).
HPLC (t, %): 9.06 min, 100%.
MS(ESI) *m*/*z* = 340 (M+1)
¹H NMR (400 MHz, ppm, CDCl₃): 2.31 (2H, m), 3.17 (1H, m), 3.43 (2H, m), 3.54 (1H, m), 3.67 (1H, m), 5.17 (2H,s), 6.50 (1H, m), 6.61 (1H, t, J = 9 Hz), 6.90 (1H, m), 7.38 (5H, m)

### d) N-{(5S)-3-[3-fluoro-4-(3-cyanopyrrolidin-1-yl)phenyl]-2-oxo-5-oxazolidinyl methyl}acetamide

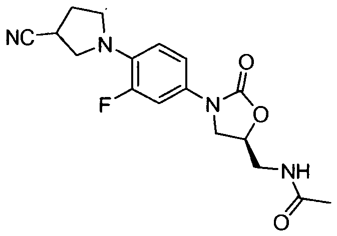

To a solution of 175 mg of 3-fluoro-4-[3-cyanopyrrolidinyl]-phenylcarbamic acid benzyl ester in 350 µL of dimethylformamide (DMF) was added 1.5 mL of a solution of 1M of lithium *tert*-butoxide in tetrahydrofuran (THF) and stirred at room temperature for 30 minutes. 42 µL of methanol and a solution of 246 mg of (*S*)-*N*-(3-bromo-2-acetoxypropyl)acetamide in 350 µL of DMF were added and allowed to stand at room temperature for two days at which point HPLC showed a 30% conversion. The same amount of lithium *tert*-butoxide in THF, methanol and (*S*)-*N*-(3-bromo-2-acetoxypropyl)acetamide were added and reaction mixture stirred at room temperature for two days more. Saturated aqueous ammonium chloride was added to the reaction solution and the separated organic layer was washed with water, brine and dried over anhydrous magnesium sulfate. The solvent was evaporated and the residue was purified by silica gel column chromatography (DCM, methanol in increasing polarity) to afford 113 mg of the title compound (Yield = 63%).
HPLC (t, %): 6.58 min, 100%.
MS(ESI) *m*/*z* = 347 (M+1)
¹H NMR (400 MHz, ppm, DMSO): 2.16 (1H, m), 2.30 (1H, m), 3.38 (4H, m), 3.66 (1H, dd, J = 6.8, 8.8 Hz), 4.05 (1H, t, J = 9 Hz), 4.67 (1H, m), 6.83 (1H, t, J = 9 Hz), 7.11 (1H, dd, J = 2.4, 9 Hz), 7.43 (1H, dd, J = 2.8, 16 Hz)

### Example 2: N-{(5S)-3-[3,5-difluoro-4-(3-cyanopyrrolidin-1-yl)phenyl]-2-oxo-5-oxazolidinylmethyl}acetamide

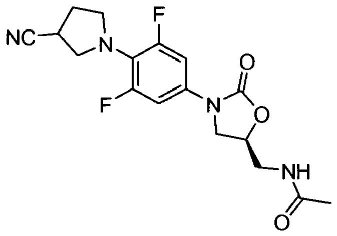

It was obtained in a similar way than compound of Example 1, starting from alkylation of 3, 4, 5-trifluoronitrobenzene with 3-carbonitrile pyrrolidine.
HPLC (t, %): 6.94 min, 92%.
MS(ESI) *m*/*z* = 365 (M+1)
¹H NMR (400 MHz, ppm, CDCl₃): 1.82 (3H, s), 2.25 (2H, m), 3.13 (1H, q, J = 7.2 Hz), 3.58 (7H, m), 3.96 (1H, t, J = 9.2 Hz), 4.75 (1H, m), 6.04 (1H, NH), 7.06 (2H, d, J = 10.8 Hz)

### Example 3: Pharmaceutical compositions

The following illustrate representative pharmaceutical compositions containing a compound of formula (I) or a pharmaceutically acceptable salt thereof for antimicrobial use in humans or animals:

| Tablet 1 | mg/tablet |
|---|---|
| Active ingredient | 100 |
| Lactose | 179 |
| Croscarmellose sodium | 12 |
| Polyvinylpyrrolidone | 6 |
| Magnesium stearate | 3 |

| Tablet 2 | mg/tablet |
|---|---|
| Active ingredient | 50 |
| Lactose | 229 |
| Croscarmellose sodium | 12 |
| Polyvinylpyrrolidone | 6 |
| Magnesium stearate | 3 |

| Tablet 3 | mg/tablet |
|---|---|
| Active ingredient | 1 |
| Lactose | 92 |
| Croscarmellose sodium | 4 |
| Polyvinylpyrrolidone | 2 |
| Magnesium stearate | 1 |

| Capsule | mg/capsule |
|---|---|
| Active ingredient | 10 |
| Lactose | 389 |
| Croscarmellose sodium | 100 |
| Magnesium stearate | 1 |

| Injection | 50 mg/mL |
|---|---|
| Active ingredient | 5.0% w/v |
| Isotonic aqueous solution | to 100% |

Buffers, pharmaceutically acceptable co-solvents such as polyethylene glycol, polypropylene glycol, glycerol or ethanol or complexing agents, may be used to aid formulation.

The above formulations may be prepared by well-known conventional procedures in the pharmaceutical art. The tablets 1-3 may be enteric coated by conventional means, for example to provide a coating of cellulose acetate phthalate.

### Example 4: Antibacterial activity compared with linezolid

MICs were determined by using a standard micro dilution method according to The National Committee for Clinical Laboratory Standards (NCCLS), 5^{th} Approved standard M7-A5, 2001, Wayne, PA, USA. All compounds were tested against Gram-positive and Gram-negative bacteria showing relevant different susceptibility and resistance specifications. The used micro organisms were selected from laboratory reference bacteria and from clinical isolates. The tested concentrations were double dilutions from 0.06 µg/mL to 128 µg/mL in 96-well micro titter plates.

MICs were determined in the *Brucella* Blood medium supplemented for the anaerobic strains, and in the Mueller-Hinton culture medium (cation-adjusted) for the aerobic bacteria.

The tested compounds were dissolved in DMSO, and were diluted as far as 2560 µg/mL with the different media according to the specific requirements for each group of strains. The 96-well sealed micro titter plates containing bacteria were incubated in different laboratory conditions depending on the nature of the microorganism. Thus, the aerobic bacteria were incubated during 16-24 h at 35°C and the so-called fastidious bacteria, such as *M. catarrhalis* and *S. pneumoniae,* during 20-24h at 35°C in a microaerobiotic atmosphere containing 5% CO₂ (Anaerocult C, MERCK). The results of these tests are given in Table 1.

**Table 1**

| | Ex. 1 | Ex. 2 | Linezolid |
|---|---|---|---|
| *S. aureus* ATCC25923 MS | 0.5 | 0.25 | 1.00 |
| *S. pneumoniae* ATCC49619 PR | 0.25 | 0.125 | 1.00 |
| *E. faecium* ATCC51559 MDR | 0.25 | 0.125 | 0.50 |
| *S. aureus* LNZ-R 432 | 4 | 64 | 16-32 |
| *H. influenzae* ATCC49247 | 4 | 2 | 16.00 |
| *B. fragilis* sp. fragilis ATCC25285 | 0.5 | 0.125 | 2.00 |
| *Moraxella catarrhalis* HCl-78 | 2 | 0.25 | 2.00 |
| *E. faecium* LNZ-R LR-4 | 8 | 4 | 64.00 |

MIC values of table 1 show that less concentration of compounds of example 1 and 2 is required to inhibit the bacterial grown than for linezolid. Thus, the previous results show that the compounds of the present invention are more active than against linezolid-resistant strains.

### Comparative Example 5: (S)-N-[[3-[4-(3-hydroxypyrrolidinyl)-3-fluorophenyl]-2-oxo-5-oxazolidinyl]methyl]acetamide

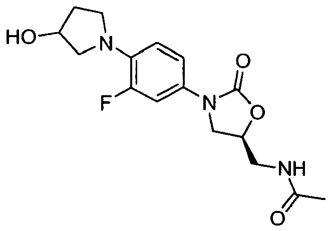

It was obtained according to the process disclosed in the example 10 page 50 of the International patent application WO 9613502.

### Example 6: Antibacterial activity compared with the compound of comparative Example 5

This example compares the antibacterial activity against linezolid-resistant strains and Gram positive pathogenic respiratory bacteria of the compound of example 1 of the present invention with the reference compound of example 5. Compound of example 5 is the structurally closest oxazolidinone of the state of the art, where the pyrrolidine ring contains a hydroxyl moiety instead of the cyano group.

In order to compare the antibacterial activity of both compounds, MICs were determined by the same methods described in example 4 and the tested samples were also prepared as shown in example 4.

The results are summarized in Table 2.

**Table 2**

| | Ex. 1 (µg/mL) | Ex. 5 (µg/mL) |
|---|---|---|
| *S. aureus* clinical strain cfr. methylase | 4 | 16 |
| *S. aureus* 1199 | 1 | 2 |
| *S. aureus* 199B | 1 | 2 |
| MRSE | 0.25 | 1 |
| MRSE-128 | 8 | 32 |
| *S. Haemolyticus* | 8 | 32 |
| *E. faecalis* ECFL-1 | 0.125 | 2 |
| *E. faecalis* ECFL-1-128 | 32 | 64 |
| *E. faecalis* ECFL-2 | 1 | 4 |
| *E. faecium* ECFM-1 | 0.5 | 2 |
| *E. faecium* ECFM-1-128 | 32 | 64 |
| *E. faecium* ECFM-2 | 0.5 | 2 |
| Geom. Mean MIC *Staphyl.* | 0.4 | 1.2 |
| Geom. Mean MIC *Enteroc.* | 0.3 | 1.7 |
| Geom. Mean MIC Gram+ | 0.3 | 1.4 |
| *S. aureus* ATCC33591 *in vivo* | 0.25 | 2 |
| *S. pneumoniae* ATCC6303 *in vivo* | 0.25 | 0.5 |
| *S. aureus* ATCC25923 MS | 0.5 | 2 |
| *S. aureus* ATCC43300 MR | 0.5 | 1 |
| *S. aureus* 1199B NorA overE | 0.5 | 1 |
| *S. epidermidis* ATCC12228 MR | 0.25 | 1 |
| *S. pneumoniae* ATCC49619 PR | 0.25 | 1 |
| *E. faecalis* ATCC29212 | 0.25 | 2 |
| *E. faecalis* ATCC51575 MDR | 0.25 | 2 |
| *E. faecium* ATCC10541 | 0.25 | 2 |
| *E. faecium* ATCC51559 MDR | 0.25 | 1 |
| *E. faecium* LNZ-R LR-4 | 8 | 32 |
| *E. coli* TG1 | 32 | 64 |
| *E. coli* KAM-3 | 1 | 4 |

MIC values of Table 2 show that less concentration of the compound of example 1 is required to inhibit the bacterial grown in respect to compound of example 5.

Therefore, compound of example 1 is much more effective as an antibacterial agent including linezolid-resistant strains and Gram-positive pathogenic respiratory bacteria in all antibacterial species tested.

## Claims

1. A compound of formula (I), 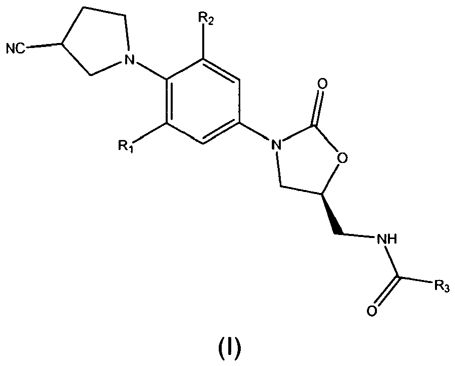 in free or pharmaceutically acceptable salt, solvate, hydrate, or enantiomeric form, wherein:
R₁ and R₂ are radicals identical or different and are independently selected from hydrogen and fluorine;
R₃ is a linear or branched (1-6C)alkyl group optionally substituted by a group selected from fluorine, hydroxy and OR₄; and
R₄ is a linear or branched (1-6C)alkyl group.

2. The compound according to claim 1, wherein R₁ is fluorine.

3. The compound according to claim 1, wherein R₂ is selected from fluorine and hydrogen.

4. The compound according to claim 1, wherein R₃ is methyl.

5. The compound as claimed in any of the preceding claims, which is selected from the group consisting of:
*N*-{(5S)-3-[3-fluoro-4-(3-cyanopyrrolidin-1-yl)phenyl]-2-oxo-5-oxazolidinylmethyl}acetamide;
*N*-{(5S)-3-[3,5-difluoro-4-(3-cyanopyrrolidin-1-yl)phenyl]-2-oxo-5-oxazolidinylmethyl}acetamide;
*N*-{(5S)-3-[3-fluoro-4-(3(R)-cyanopyrrolidin-1-yl)phenyl]-2-oxo-5-oxazolidinylmethyl}acetamide;
*N*-{(5S)-3-[3,5-difluoro-4-(3(R)-cyanopyrrolidin-1-yl)phenyl]-2-oxo-5-oxazolidinylmethyl}acetamide;
*N*-{(5S)-3-[3-fluoro-4-(3(S)-cyanopyrrolidin-1-yl)phenyl]-2-oxo-5-oxazolidinylmethyl}acetamide; and
*N*-{(5S)-3-[3,5-difluoro-4-(3(S)-cyanopyrrolidin-1-yl)phenyl]-2-oxo-5-oxazolidinylmethyl}acetamide.

6. A process for preparing a compound of formula (I) in free or pharmaceutically acceptable salt, solvate, hydrate, or enantiomeric form that comprises:
(i) reacting an intermediate of formula (II), 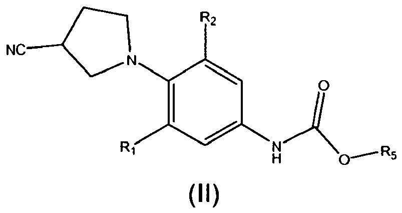 wherein R₁ and R₂ are as defined above and R₅ is selected from linear or branched (1-6C)alkyl and benzyl optionally phenyl-substituted by up to three linear or branched (1-6C)alkyl groups, with an intermediate of formula (III), 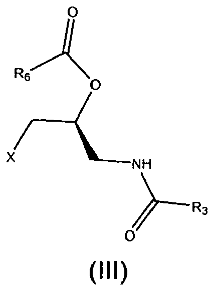 wherein R₃ is as defined above, R₆ is a linear or branched (1-6C)alkyl group, and X is a halogen atom; and
(ii) recovering the resultant compound of formula (I) in free or pharmaceutically acceptable salt, solvate, hydrate, or enantiomeric form.

7. The process of claim 6 wherein R₅ is benzyl, R₆ is methyl and X is bromine.

8. A pharmaceutical composition comprising a therapeutically effective amount of the compound of formula (I) as defined in any of the claims 1 to 5, together with the appropriate amounts of pharmaceutical excipients or carriers.

9. A compound of formula (I) as defined in any of the claims 1 to 5 for use as a medicament.

10. Use of a compound of formula (I) as defined in any of the claims 1 to 5 for the manufacture of a medicament for the treatment of bacterial infections in an animal or human.

11. A compound of formula (I) as defined in any of the claims 1 to 5 for use in the treatment of bacterial infections.

12. The compound for use of formula (I) according to claim 11, wherein the bacterial infection is an infection produced by linezolid-resistant strain

13. The compound for use of formula (I) according to claim 11, wherein the bacterial infection is an infection produced by Gram-positive pathogenic respiratory bacteria

## Patentansprüche

1. Verbindung mit der Formel (I), 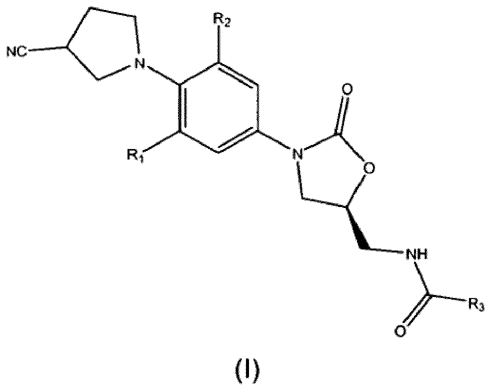 in freier Form oder in Form eines pharmazeutisch verträglichen Salzes, Solvats, Hydrats, oder Enantiomers, worin:
R₁ und R₂ gleich- oder verschiedenartige Radikale sind und unabhängig voneinander aus Wasserstoff und Fluor ausgewählt werden;
R₃ eine verzweigte oder unverzweigte (1-6C)Alkylgruppe ist, die gegebenenfalls mit einem Rest substituiert ist, der aus Fluor, Hydroxy und OR₄ ausgewählt wird; und
R₄ eine verzweigte oder unverzweigte (1-6C)Alkylgruppe ist.

2. Verbindung nach Anspruch 1, worin R₁ Fluor ist.

3. Verbindung nach Anspruch 1, worin R₂ aus Fluor und Wasserstoff ausgewählt wird.

4. Verbindung nach Anspruch 1, worin R₃ Methyl ist.

5. Verbindung nach einem der vorstehenden Ansprüche, die aus der folgenden Gruppe ausgewählt wird:
N-{(5S)-3-[3-fluor-4-(3-cyanpyrrolidin-1-yl)phenyl]-2-oxo-5-oxazolidinylmethyl}Acetamid;
N-{(5S)-3-[3,5-difluor-4-(3-cyanpyrrolidin-1-yl)phenyl]-2-oxo-5-oxazolidinylmethyl}Acetamid;
N-{(5S)-3-[3-fluor-4-(3(R)-cyanpyrrolidin-1-yl)phenyl]-2-oxo-5-oxazolidinylmethyl}Acetamid;
N-{(5S)-3-[3,5-difluor-4-(3(R)-cyanpyrrolidin-1-yl)phenyl]-2-oxo-5-oxazolidinylmethyl}Acetamid;
N-{(5S)-3-[3-fluor-4-(3(S)-cyanpyrrolidin-1-yl)phenyl]-2-oxo-5-oxazolidinylmethyl}Acetamid; und
N-{(5S)-3-[3,5-difluor-4-(3(S)-cyanpyrrolidin-1-yl)phenyl]-2-oxo-5-oxazolidinylmethyl}Acetamid.

6. Verfahren zur Herstellung einer Verbindung mit der Formel (I) in freier Form oder in Form eines pharmazeutisch verträglichen Salzes, Solvates, Hydrates oder Enantiomeres, bei dem
(i) ein Zwischenprodukt mit der Formel (II), 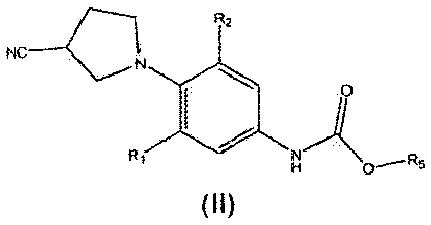 worin R₁ und R₂ der obigen Definition entsprechen und R₅ aus der Gruppe aus verzweigtem und unverzweigtem (1-6C)Alkyl und Benzyl, das gegebenenfalls mit bis zu drei verzweigten oder unverzweigten (1-6C)Alkylgruppen phenyl-substituiert ist, ausgewählt wird, mit einem Zwischenprodukt mit der Formel (III), 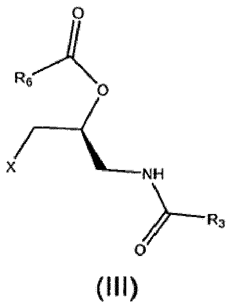 worin R₃ der obigen Definition entspricht, R₆ eine verzweigte oder unverzweigte (1-6C)Alkylgruppe ist, und X ein Halogenatom ist, zur Reaktion gebracht wird; und
(ii) die dabei entstandene Verbindung der Formel (I) in freier Form oder in Form eines pharmazeutisch verträglichen Salzes, Solvates, Hydrates oder Enantiomers abgetrennt wird.

7. Verfahren nach Anspruch 6 worin R₅ Benzyl ist, R₆ Methyl und X Brom ist.

8. Pharmazeutische Zusammensetzung, die eine therapeutisch wirksame Menge der Verbindung mit der Formel (I) nach einem der Ansprüche 1 bis 5 zusammen mit angemessenen Mengen pharmazeutisch verträglicher Hilfs- und Trägerstoffe enthält.

9. Verbindung mit der Formel (I) nach einem der Ansprüche 1 bis 5 zur Verwendung als Medikament.

10. Verwendung einer Verbindung mit der Formel (I) nach einem der Ansprüche 1 bis 5 zur Herstellung eines Medikaments zur Behandlung bakterieller Infektionen bei einem Tier oder Menschen.

11. Verbindung mit der Formel (I) nach einem der Ansprüche 1 bis 5 zur Verwendung bei der Behandlung von bakteriellen Infektionen.

12. Verbindung mit der Formel (I) zur Verwendung nach Anspruch 11, wobei die bakterielle Infektion eine Infektion ist, die durch einen Linezolid-resistenten Bakterienstamm verursacht wurde.

13. Verbindung mit der Formel (I) zur Verwendung nach Anspruch 11, wobei die bakterielle Infektion eine Infektion ist, die durch Gram-positive pathogene Bakterien des Atmungssystems verursacht wurde.

## Revendications

1. Composé de formule (I), 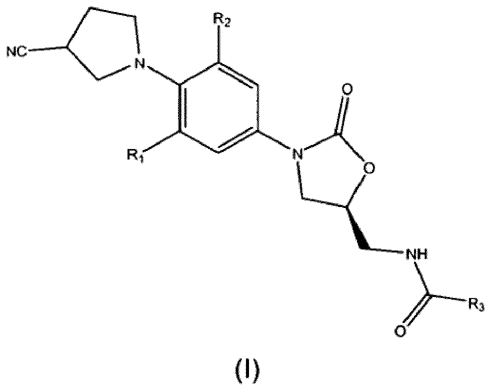 sous forme libre ou de sel, solvate, hydrate ou énantiomère acceptable sur le plan pharmaceutique, dans lequel :
R₁ et R₂ sont des radicaux identiques ou différents et sont indépendamment choisis parmi l'hydrogène et le fluor ;
R₃ est un groupe alkyle comportant 1 à 6 atomes de carbone, linéaire ou ramifié,
éventuellement substitué par un groupe choisi parmi fluor, hydroxy et OR₄ ; et
R₄ est un groupe alkyle comportant 1 à 6 atomes de carbone, linéaire ou ramifié.

2. Composé selon la revendication 1, dans lequel R₁ est le fluor ;

3. Composé selon la revendication 1, dans lequel R₂ est choisi parmi le fluor et l'hydrogène.

4. Composé selon la revendication 1, dans lequel R₃ est un groupe méthyle.

5. Composé selon l'une quelconque des revendications précédentes, qui est choisi dans le groupe constitué de :
*N*-{(5S)-3-[3-fluoro-4-(3-cyanopyrrolidin-1-yl)phényl]-2-oxo-5-oxazolidinylméthyl}acétamide ;
*N*-{(5S)-3-[3,5-difluoro-4-(3-cyanopyrrolidin-1-yl)phényl]-2-oxo-5-oxazolidinylméthyl}acétamide ;
*N*-{(5S)-3-[3-fluoro-4-(3(R)-cyanopyrrolidin-1-yl)phényl]-2-oxo-5-oxazolidinylméthyl}acétamide ;
*N*-{(5S)-3-[3,5-difluoro-4-(3(R)-cyanopyrrolidin-1-yl)phényl]-2-oxo-5-oxazolidinylméthyl}acétamide ;
*N*-{(5S)-3-[3-fluoro-4-(3(S)-cyanopyrrolidin-1-yl)phényl]-2-oxo-5-oxazolidinylméthyl}acétamide ; et
*N*-{(5S)-3-[3,5-difluoro-4-(3(S)-cyanopyrrolidin-1-yl)phényl]-2-oxo-5-oxazolidinylméthyl}acétamide.

6. Procédé pour préparer un composé de formule (I) sous forme libre ou de sel, solvate, hydrate ou énantiomère acceptable sur le plan pharmaceutique, qui comprend :
(i) la réaction d'un intermédiaire de formule (II), 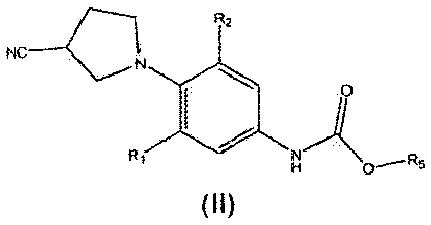 dans laquelle R₁ et R₂ sont tels que définis précédemment et R₅ est choisi parmi un groupe alkyle comportant 1 à 6 atomes de carbone, linéaire ou ramifié et un benzyle éventuellement substitué sur le phényle par jusqu'à trois groupes alkyle comportant 1 à 6 atomes de carbone, linéaires ou ramifiés, avec un intermédiaire de la formule (III), 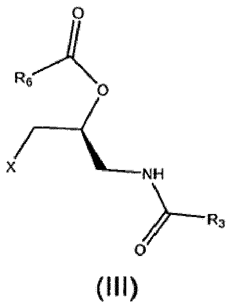 dans laquelle R₃ est tel que défini précédemment, R₆ est un groupe alkyle comportant 1 à 6 atomes de carbone, linéaire ou ramifié, et X est un atome d'halogène ; et
(ii) la récupération du composé résultant de formule (I) sous forme libre ou de sel, solvate, hydrate ou énantiomère acceptable sur le plan pharmaceutique.

7. Procédé selon la revendication 6, dans lequel R₅ est benzyle, R₆ est méthyle et X est brome.

8. Composition pharmaceutique comprenant une quantité efficace sur le plan thérapeutique du composé de formule (I) tel que défini dans l'une quelconque des revendications 1 à 5, conjointement avec les quantités appropriées d'excipients ou vecteurs pharmaceutiques.

9. Composé de formule (I) selon l'une quelconque des revendications 1 à 5 pour une utilisation en tant que médicament.

10. Utilisation d'un composé de formule (I) selon l'une quelconque des revendications 1 à 5 pour la fabrication d'un médicament pour le traitement d'infections bactériennes chez un animal ou un humain.

11. Composé de formule (I) selon l'une quelconque des revendications 1 à 5, destiné à être utilisé dans le traitement d'infections bactériennes

12. Composé utilisable de la formule (I) selon la revendication 11, dans lequel l'infection bactérienne est une infection produite par une souche résistante au linézolide.

13. Composé utilisable de la formule (I) selon la revendication 11, dans lequel l'infection bactérienne est une infection produite par bactérie respiratoire pathogène à Gram positif.
